(19) Europäisches Patentamt
European Patent Office
Office européen des brevets

(11) Publication number: **0 088 395**
**B1**

(12) # EUROPEAN PATENT SPECIFICATION

(45) Date of publication of patent specification: **16.07.86**

(51) Int. Cl.$^4$: **C 07 D 241/12,** C 07 D 241/24

(21) Application number: **83102138.1**

(22) Date of filing: **04.03.83**

(54) **A process for the preparation of pyrazine derivatives.**

(30) Priority: **08.03.82 GB 8206772**

(43) Date of publication of application:
**14.09.83 Bulletin 83/37**

(45) Publication of the grant of the patent:
**16.07.86 Bulletin 86/29**

(84) Designated Contracting States:
**AT BE CH DE FR GB IT LI NL SE**

(56) References cited:
**GB-A-1 361 967**

**G.B. BARLIN:"THE PYRAZINES", vol. 41, January 1982, John Wiley, chapter III: "Primary Syntheses of Pyrazines N-Oxides", Brisbane (AU);**

(73) Proprietor: **FARMITALIA CARLO ERBA S.p.A.**
**Via Carlo Imbonati n.24**
**I-20159 Milan (IT)**

(72) Inventor: **Ribaldone, Giuseppe**
**Via Carlo Cattaneo, 37**
**Gallarate (VA) (IT)**
Inventor: **Felicioli, Maria Giuseppina**
**Viale Allegra, 11**
**Novara (IT)**
Inventor: **Santini, Claudio**
**Via Venezia, 1**
**Novara (IT)**
Inventor: **Agnes, Giovanni**
**Via Corridoni, 22**
**Novara (IT)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for the preparation of pyrazine derivatives of the general formula I

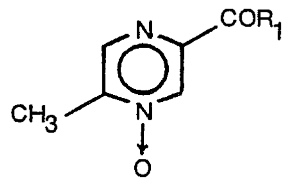

I

wherein $R_1$ represents a hydoxyl group, an alkoxy group with 1 to 6 carbon atoms, or a group of formula $NR_2R_3$; wherein both $R_2$ and $R_3$ are independently hydrogen, an alkyl group with 1 to 6 carbon atoms, or they are linked together to form a ring which may comprise other heteroatoms, such as O, N and S.

The meaning of "alkoxy group with 1 to 6 carbon atoms" as used in this specification comprises methoxy, ethoxy, n- and iso-propoxy, n-, iso-, sec.- and tert.-butoxy as well as isomers of pentoxy and hexoxy.

The meaning of "alkyl group with 1 to 6 carbon atoms" as used in this specification comprises methyl, ethyl, n- and iso-propyl as well as the isomeric forms of butyl, pentyl and hexyl. In particular, the method according to the invention is useful for the preparation of 5-methylpyrazine-2-carboxylic-4-oxido acid ($R_1$ = OH).

Compounds of formula I as above defined have been recognized as having hypolipaemic or hypoglycaemic activity as is demonstrated in GB—PS 1,361,967.

Said patent also provides a method for preparing compounds of formula I which comprises the oxidation of a compound of the following formula:

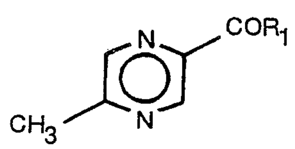

wherein $R_1$ is as above defined, by means of hydrogen peroxide. While this previously described process may be satisfactory to obtain small quantities, the use of a dangerous oxidizing reagent, such as hydrogen peroxide, is not suitable for industrial scale.

Now, the applicant surprisingly found a safe and new method for preparing compounds of formula I on a large scale without the use of an oxidizing reagent.

Pyrazine derivatives of the general formula (I), wherein $R_1$ is as defined above, can be prepared according to this invention following a precedure which comprises condensation of a compound of the general formula (II)

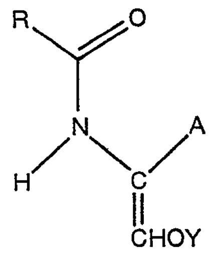

(II)

wherein R represents hydrogen, or an alkyl or alkoxy group with 1 to 6 carbon atoms, Y represents hydrogen, sodium, potassium or lithium, and A is a nitrile group or a substituent of the formula $COR_1$, in which $R_1$ has the same significance as defined above, with pyruvic aldehyde oxime of formula (III)

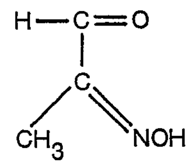

(III)

which can be used in the form of its acetal derivative (IV)

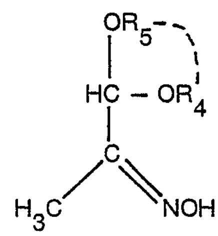

(IV)

wherein $R_4$ and $R_5$ are independently hydrogen, an alkyl group with 1 to 6 carbon atoms, or they may be linked together to form a cyclic acetal of the formula IV; and, if A is a nitrile group, hydrolyzation of the reaction product; and, if desired, convertion of a compound of formula I produced in this way into another compound of formula I by a method known *per se.*

Acetal derivatives of formula (IV) may possibly be generated "in situ" by the reaction between a pyruvic aldehyde di-alkyl or cyclic acetal (V)

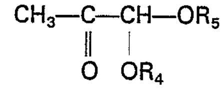

wherein $R_4$ and $R_5$ are as defined above, with hydroxylamine according to methods known to experts in this field.

Compounds of formula (II) have been prepared according to the methods described by Ellis V. Brown, "Chemistry of penicillin", Princetown University Press 1949, pages 473—534, or by extensions or modifications of this method which are quite obvious to experts in the field.

It should be noted that such compounds can be

indicated not only be formula (II) (adopted in the present specification according to E. V. Brown), but also by formula VI, wherein R, A and Y have the same meaning as described above:

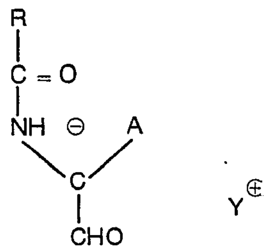

(V)

In the fact, II and VI constitute limit formulae and it is presumed that both forms are present together and in equilibrium, and suitable for indicating one and the same compound.

Compounds of formula III and IV have been prepared according to conventional methods (see e.g. G. T. Newbold, W. Sharp and F. S. Spring, J. Chem. Soc., 1951, 2679).

The reaction between compounds of formula II with compounds of formula III and/or IV, to which the present invention refers, is preferably carried out in a suitable solvent in the presence of organic or inorganic acids at a temperature falling between 15°C and the boiling point of the chosen solvent.

The preferable acids are hydrochloric, hydrobromic, hydroiodic, sulphuric, nitric, p-toluenemethanesulphonic, formic, phosphoric, methanesulphonic, trifluoroacetic and trifluoromethanesulphonic acid.

The solvent is preferably chosen from among $R_7OH$ alcohols, $R_7OR_6$ ethers,

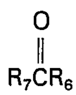 ketones,

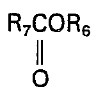 esters

or organic acids, in which both $R_7$ and $R_6$ are independently hydrogen and branched, linear or cyclic alkyl residues containing 1 to 6 carbon atoms, and where both are present, they can be jointed together to form either a cyclic ether, a cyclic ketone or a lactone.

The reaction according to the invention is carried out preferably using equimolecular quantities of II and III, or II and IV, or II with a mixture of pyruvaldehyde V and hydroxylamine capable of forming III.

The dilution proportions between II (or III) and the solvent preferably range from 1:10 and 1:100, but even higher dilutions may be used.

When A in formula III is equal to nitrile, the compounds of formula I can be prepared by hydrolysing the product obtained by condensation of II with III and/or IV according to methods known per se.

A compound of formula I prepared according to the invention may be converted into another compound of formula I by methods known *per se*. The conversion of a compound of formula I wherein $R_1$ is an alkoxy group or a $NR_2R_3$ group into a compound of formula I wherein $R_1$ is hydroxy group may be performed for example by saponification with an aqueous alkali or by hydrolysis.

The examples given below are merely indicative.

### Example 1

The following components are placed in the reactor: 50 ml dioxane, 2 g methyl α-formyl-N-formyl glycinate, sodium salt (II, wherein R = H, $R_1 = OCH_3$, Y = Na), 1.6 g methyl glyoxime-dimethyl acetal (IV, wherein $R_4 = R_5 = CH_3$) and 2 ml 35% HCl solution.

The whole is stirred for 5 hours at a temperature of 45°C to 50°C; the temperature is maintained thermostatically.

The white solid which forms (NaCl) is filtered off. The solvent is eliminated under vacuum and the residue is taken up and recrystallized from methanol, obtaining 1.1 g methyl-4-oxide 5-methyl-pyrazine-2-carboxylate.

Yield: 56%

Melting point: 146 to 147°C

### Example 2

25 ml isopropyl alcohol, 1 g ethyl α-formyl-N-formyl glycinate, sodium salt (II, wherein R = H, $R_1 = OC_2H_5$, Y = Na), 0.75 g methylglyoxime dimethylacetal (IV, $R_4 = R_5 = CH_3$) and 1 ml 35% HCl solution are placed in the reactor.

The mixture is stirred for 5 hours at 60°C and the solvent is then evaporated under vacuum. 25 ml of ethanol are added and the whole is allowed to stand for 10 hours.

0.48 g (48% yield) of ethyl-4-oxide 5-methyl-pyrazine-2-carboxlate are obtained.

### Example 3

5 g ethyl α-formyl-N-formyl glycinate, sodium salt (II, R = H, $R_1$ = OEt, Y = Na) is introduced into the reaction flask, along with 3.7 g methyl glyoxime dimethyl acetal (IV, wherein $R_4 = R_5 = CH_3$), 125 ml isopropyl alcohol and 5 ml conc. HCl (35%).

The mixture is stirred for 6 hours at 60°C.

The NaCl formed is filtered off.

Cooled to 0°C and saturated with gaseous $NH_3$, the filtrate gives after 10 hours a precipitate of 1.9 g of 5-methylpyrazine-2-carboxamide-4-oxide, which is isolated and in equivalent to a yield of 45%.

Melting point: 209 to 210°C

### Example 4

1 g ethyl α-formyl-N-formyl glycinate, sodium salt (II, wherein R = H, $R_1 = OC_2H_5$, Y = Na), 0.75 g methylglyoxime dimethylacetal (IV, wherein

$R_4 = R_5 = CH_3$) and 25 ml 85% HCOOH in water, and placed in a reaction flask.

The whole is stirred for 5 hours at 50°C.

The formic acid and water are distilled off under vacuum.

Ethanol (30 ml) containing 1% HCl is added to the residue.

The mixture is then refluxed for 3 hours. 0.33 g of ethyl-4-oxide 5-methyl-pyrazine-2-carboxylate are obtained (33% yield).

Example 5

1 g ethyl α-formyl-N-formyl-glycinate, sodium salt (II, R = H, $R_1 = OC_2H_5$, Y = Na), 0.75 g methylglyoxime dimethylacetal (IV, wherein $R_4 = R_5 = CH_3$), 25 ml ethyl acetate and 1 ml conc. HCl (35%), are introduced into a reaction flask.

The mass is stirred for 8 hours at 50 to 55°C. On operating as described in example 2, 0.45 g of ethyl-4-oxide 5-methyl-pyrazine-2-carboxylate are obtained, equal to a yield of 45%.

Example 6

Operating according to the method and quantities given for the first part of example 3, and replacing the conc. HCl with 2 g para-toluenesulphonic acid, 0.37 g (= 37% yield) of ethyl-4-oxide 5-methyl-pyrazine-2-caboxylate are obtained.

Example 7

Operating according to the quantities and method established in example 5, but replacing the ethyl acetate with the same quantity (25 ml) of acetone, 0.36 g of ethyl 4-oxide 5-methyl-pyrazine-2-carboxylate are obtained (36% yield).

Example 8

1 g ethyl α-formyl-N-formyl glycinate, sodium salt (II, wherein R = H, $R_1$ = OEt, Y = Na), 0.5 g pyruvic aldehyde oxime (III), 1 ml conc. HCl (35%) and 25 ml isopropyl alcohol, are introduced into the reaction flask.

The mass is kept at 60°C for 8 hours. On operating as described in example 5, 0.31 g of ethyl 4-oxide 5-methyl-pyrazine-2-carboxylate are obtained.

Example 9

4.2 g of pyruvaldehyde dimethylacetal (V, $R_4 = R_5 = CH_3$) are placed in the reaction flask.

2.45 g of $NH_2OH.HCl$, dissolved in 10 ml of $H_2O$ and brought to pH 7 with $Na_2CO_3$, are then added to the flask. This operation is carried out at 5 to 10°C. 120 ml of 98% HCOOH, 5.6 g of ethyl α-formyl-N-foryl glycinate, sodium salt (II, R = H, $R_1$ = OEt, Y = Na) are added after 30 minutes. The mixture is stirred for 5 hours at 50°C. On operating as described in example 4, 1.4 g of ethyl-4-oxide 5-methyl-pyrazine-2-carboxylate are obtained. 25% yield.

Example 10

1 g ethyl α-formyl-N-carbethoxyglycinate, sodium salt (II, wherein $R = R_1 = OC_2H_5$, Y = Na), 0.6 g methylglyoxime dimethylacetal (IV, wherein $R_4 = R_5 = CH_3$), 25 ml isopropyl alcohol and 0.8 ml HCl (35%) are placed in the reaction flask.

The mass is then refluxed for 10 hours with stirring.

0.21 g of ethyl-4-oxide 5-methyl-pyrazine-2-carboxylate, equivalent to a yield off 27.3%, are obtained after the usual treatment.

Example 11

8 g of methyl α-formyl-N-acetylglycinate, sodium salt (II wherein $R = CH_3$, $R_1 = OCH_3$, Y = Na), methylglyoxime dimethylacetal (IV wherein $R_4 = R_5 = CH_3$), 200 ml of isopropyl alcohol and 7.5 ml of 35% HCl are placed in the reaction flask.

The mass is stirred for 5 hours at 60°C.

3 g of methyl-4-oxide 5-methyl-pyrazine-2-carboxylate, equivalent to a yield of 40%, are obtained after the usual treatment.

**Claims**

1. A process for the preparation of a pyrazine derivative of the formula I:

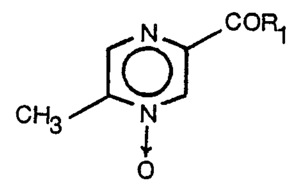

I

wherein $R_1$ represents hydroxy, $C_1$—$C_6$ alkoxy or a group of the formula $NR_2R_3$, in which $R_2$ and $R_3$ are independently hydrogen or $C_1$—$C_6$ alkyl or, together with the nitrogen atom to which they are linked, they may form a ring which may contain one or more additional heteroatoms; which process comprises reacting a compound of formula II:

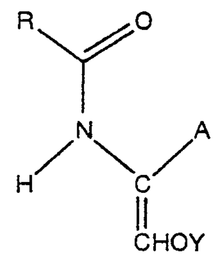

(II)

wherein R represents hydrogen, $C_1$—$C_6$ alkyl or $C_1$—$C_6$ alkoxy, Y represents hydrogen, sodium, lithium or potassium, and A is a nitrile group or a substituent of the formula $COR_1$, wherein $R_1$ is as defined above, either with pyruvic aldehyde oxime of formula III:

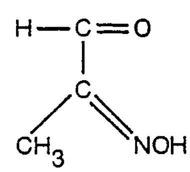

(III)

or with a pyruvic aldehyde oxime acetal derivative of formula IV:

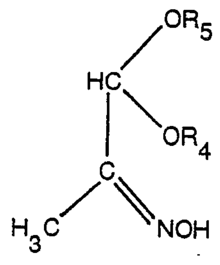

(IV)

wherein $R_4$ and $R_5$ are independently hydrogen or $C_1$—$C_6$ alkyl or may be linked together so that the compound of formula IV is a cyclic acetal; and, when A is a nitrile group, hydrolyzing the reactions product; and, if desired, converting a compound of formula I so produced into another compound of formula I by a method known *per se*.

2. A process according to claim 1 wherein the reaction is between a compound of formula II and an acetal derivative of formula IV, the acetal derivative being generated *in situ* by reacting hydroxylamine with a pyruvic aldehyde derivative of formula V:

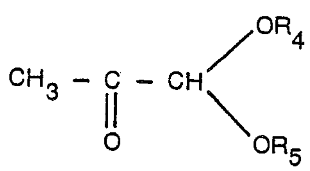

(V)

wherein $R_4$ and $R_5$ are as defined in claim 1.

3. A process according to claim 1 wherein the reaction is carried out in a suitable solvent, in the presence of an organic or inorganic acid, at a temperature from 15°C to the boiling point of the solvent.

4. A process according to claim 3 wherein the acid is hydrochloric, hydrobromic, hydroiodic, sulphuric, nitric, p-toluene-sulphonic, formic, phosphoric, methanesulphonic, trifluoroacetic or trifluoromethanesulphonic acid.

5. A process according to claim 4 wherein the acid is hydrochloric acid.

6. A process according to claim 3, wherein the solvent is an alcohol of formula $R_7OH$, an ether of formula $R_7OR_6$, a ketone of formula $R_7COR_6$, an ester of formula $R_7COOR_6$ or an organic acid, $R_7$ and $R_6$ being independently hydrogen, a linear, branched or cyclic alkyl group of 1 to 6 carbon atoms or, where $R_6$ and $R_7$ are both present, they may be linked together so that the solvent is a cyclic ether, a cyclic ketone, or a lactone.

7. A process according to claim 6 wherein the solvent is dioxane.

8. A process according to claim 6 wherein the solvent is isopropyl alcohol.

9. A process according to claim 1 wherein $R_1$ represents hydroxy.

10. A process according to claim 1 wherein $R_1$ represents methoxy.

11. A process according to claim 1 wherein $R_1$ represents an amine group.

**Patentansprüche**

1. Verfahren zur Herstellung eines Pyrazin-Derivates der Formel (I):

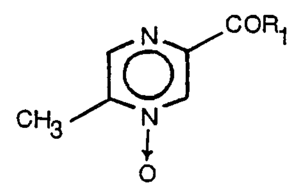

I

worin $R_1$ Hydroxy, $C_{1-6}$-Alkoxy oder eine Gruppe der Formel $NR_2R_3$ bedeutet, worin $R_2$ und $R_3$ unabhängig voneinander Wasserstoff oder $C_{1-6}$-Alkyl darstellen, oder zusammen mit dem Stickstoffatom, an welches sie gebunden sind, einen Ring bilden können, der ein oder mehrere zusätzliche Heteroatome enthält, gekennzeichnet durch Umsetzung einer Verbindung der Formel (II):

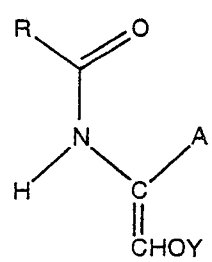

(II)

worin R Wasserstoff, $C_{1-6}$-Alkyl oder $C_{1-6}$-Alkoxy bedeutet, Y Wasserstoff, Natrium, Lithium oder Kalium darstellt und A eine Nitrilgruppe oder einen Substituenten der Formel $COR_1$ bedeutet, wobei $R_1$ die vorstehend angegebene Bedeutung hat, entweder mit Pyruvaldehydeoxim der Formel (III):

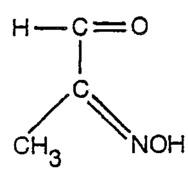

(III)

oder mit einem Pyruvaldehydehydoximacetal-Derivat der Formel (IV):

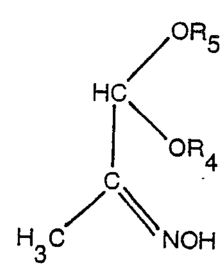

(IV)

worin $R_4$ und $R_5$ unabhängig voneinander Wasserstoff oder $C_{1-6}$-Alkyl darstellen oder miteinander verbunden sein können, so dass die Verbindung der Formel (IV) ein cyclisches Acetal darstellt; und, wenn A eine Nitrilgruppe bedeutet, Hydrolysieren des Reaktionsproduktes und — sofern dies gewünscht wird — Überführen einer Verbindung der so hergestellten Formel (I) in eine andere Verbindung der Formel (I) nach einem an sich bekannten Verfahren.

2. Verfahren nach Anspruch 1, dadurch gekennzeichnet, dass die Umsetzung zwischen einer Verbindung der Formel (II) und einem Acetal-Derivat der Formel (IV) erfolgt, wobei das Acetal-Derivat *in situ* durch Umsetzung von Hydroxylamin mit einem Pyruvaldehyd-Derivat der Formel (V):

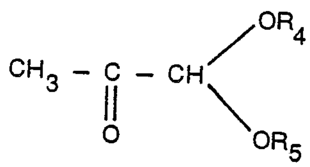
(V)

worin $R_4$ und $R_5$ die in Anspruch 1 angegebenen Bedeutungen haben, gebildet wird.

3. Verfahren nach Anspruch 1, dadurch gekennzeichnet, dass die Umsetzung in einem geeigneten Lösungsmittel in Anwesenheit einer organischen oder anorganischen Säure bei einer Temperatur von 15°C bis zum Siedepunkt des Lösungsmittels durchgeführt wird.

4. Verfahren nach Anspruch 3, dadurch gekennzeichnet, dass als Säure Salzsäure, Bromwasserstoffsäure, Jodwasserstoffsäure, Schwefelsäure, Salpetersäure, p-Toluol-sulfonsäure, Ameisensäure, Phosphorsäure, Methansulfonsäure, Trifluoressigsäure oder Trifluormethansulfonsäure verwendet wird.

5. Verfahren nach Anspruch 4, dadurch gekennzeichnet, dass die Säure Salzsäure darstellt.

6. Verfahren nach Anspruch 3, dadurch gekennzeichnet, dass das Lösungsmittel einen Alkohol der Formel $R_7OH$, einen Ether der Formel $R_7OR_6$, ein Keton der Formel $R_7COR_6$, einen Ester der Formel $R_7COOR_6$ oder eine Organische Säure darstellt, wobei $R_7$ und $R_6$ unabhängig voneinander Wasserstoff, eine lineare, verzweigte oder cyclische Alkylgruppe mit 1 bis 6 Kohlenstoffatomen bedeuten oder, wenn sowohl $R_6$ als auch $R_7$ anwesend sind, diese so miteinander verbunden sein können, dass das Lösungsmittel einen cyclischen Ether, ein cyclisches Keton oder ein Lacton darstellt.

7. Verfahren nach Anspruch 6, dadurch gekennzeichnet, dass das Lösungsmittel Dioxan darstellt.

8. Verfahren nach Anspruch 6, dadurch gekennzeichnet, dass das Lösungsmittel Isopropylalkohol darstellt.

9. Verfahren nach Anspruch 1, dadurch gekennzeichnet, dass $R_1$ Hydoxy bedeutet.

10. Verfahren nach Anspruch 1, dadurch gekennzeichnet, dass $R_1$ Methoxy bedeutet.

11. Verfahren nach Anspruch 1, dadurch gekennzeichnet, dass $R_1$ eine Amingruppe bedeutet.

**Revendications**

1. Procédé pour la préparation d'un dérivé de pyrazine de formule I:

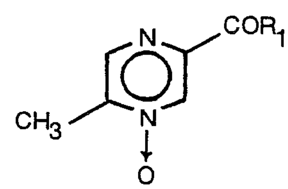
I

dans laquelle $R_1$ représente hydroxy, un group alcoxy $C_1$—$C_6$ ou un groupe de formule $NR_2R_3$, dans laquelle $R_2$ et $R_3$ représentent indépendamment l'hydrogène ou $C_1$—$C_6$ alkyle, ou ils forment avec l'atome d'azote auquel ils sont rattachés un cycle qui peut contenir un ou plusieurs hétéroatomes supplémentaires; consistant en ce que l'on fait réagir un composé de la formule II:

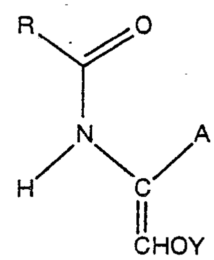
(II)

dans laquelle R représente l'hydrogène, $C_1$—$C_6$ alkyle ou $C_1$—$C_6$ alcoxy, Y représente l'hydrogène, le sodium, le lithium ou le potassium, et A est un groupe nitrile ou un substituant de formule $COR_1$, dans laquelle $R_1$ a la même signification que ci-dessus, soit avec l'oxime d'aldéhyde pyruvique de la formule III:

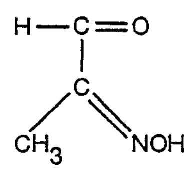
(III)

soit avec un dérivé acétal de l'oxime d'aldéhyde pyruvique de formule IV:

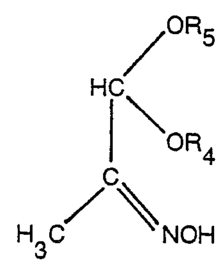
(IV)

dans laquelle $R_4$ et $R_5$ sont indépendamment l'hydrogène ou $C_1$—$C_6$ alkyle ou peuvent être liés l'un à l'autre de sorte que le composé de la formule IV est un acétal cyclique; et quand A est un groupe nitrile on hydrolyse le produit de la réaction; et, si on le désire, on convertit un composé de formule I produit de cette manière en un autre composé de formule I selon une méthode connue en soi.

2. Procédé selon la revendication 1 dans lequel la réaction a lieu entre un composé de formule II et un dérivé acétal de formule VI, le dérivé acétal étant généré in situ par réaction de l'hydroxylamine avec un dérivé de l'aldéhyde pyruvique de formule V:

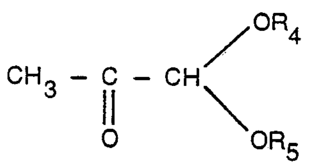

(V)

dans laquelle $R_4$ et $R_5$ ont la même signification que celle indiquée à la revendication 1.

3. Procédé selon la revendication 1 dans lequel la réaction est exécutée dans un solvant approprié, en présence d'un acide organique ou inorganique, à une température comprise entre 15°C et le point d'ébullition du solvant.

4. Procédé selon la revendication 3 dans lequel l'acide est l'acide chlorhydrique, bromhydrique, iodhydrique, sulfurique, nitrique, p-toluène sulfonique, formique, phosphorique, méthane sulfonique, trifluoroacétique ou trifluorométhanesulfonique.

5. Procédé selon la revendication 4 dans lequel l'acide est l'acide chlorhydrique.

6. Procédé selon la revendication 3 dans lequel le solvant est un alcool de formule $R_7OH$, un éther de formule $R_7OR_6$, une cétone de formule $R_7COR_6$, un ester de formule $R_7COOR_6$ ou un acide organique, $R_7$ et $R_6$ étant indépendamment de l'hydrogène, un groupe alkyle linéaire, ramifié ou cyclique avec 1 à 6 atomes de carbone ou, quand $R_6$ et $R_7$ sont tout les deux présents, ils peuvent être liés de manière que le solvant représente un éther cyclique, une cétone cyclique ou une lactone.

7. Procédé selon la revendication 6 dans lequel le solvant est la dioxanne.

8. Procédé selon la revendication 6 dans lequel le solvant est l'alcool isopropylique.

9. Procédé selon la revendication 1 dans lequel $R_1$ représente hydroxy.

10. Procédé selon la revendication 1 dans lequel $R_1$ représente méthoxy.

11. Procédé selon la revendication 1 dans lequel $R_1$ représente un groupe amine.